# EUROPÄISCHE PATENTSCHRIFT

(11) **EP 3 142 996 B1**
(45) Veröffentlichungstag und Bekanntmachung des Hinweises auf die Patenterteilung: **27.02.2019**
(21) Anmeldenummer: 15724958.2
(22) Anmeldetag: 11.05.2015
(51) Int. Cl.: C07C 201/08, C07C 201/16, C07C 209/36, C25B 7/00, B01D 17/06, B01D 17/04

(54) **VERFAHREN ZUR HERSTELLUNG VON TOLUYLENDIAMIN**
METHOD FOR THE PRODUCTION OF TOLUYLENE DIAMINE
PROCÉDÉ DE FABRICATION DE TOLUYLÈNEDIAMINE

(30) Priorität: 13.05.2014 EP 14168032
(43) Veröffentlichungstag der Anmeldung: 22.03.2017
(73) Patentinhaber: Covestro Deutschland AG, 51373 Leverkusen (DE)
(72) Erfinder: PENNEMANN, Bernd, 51467 Bergisch Gladbach (DE); MAIRATA, Antoni, 40549 Düsseldorf (DE); ZOCK, Antonia, 41462 Neuss (DE)
(74) Vertreter: Levpat
(86) Internationale Anmeldenummer: PCT/EP2015/060281
(87) Internationale Veröffentlichungsnummer: WO 2015/173161

(56) Entgegenhaltungen:
- EP-A1- 1 816 117
- EP-A1- 1 935 870
- EP-A2- 0 004 278
- WO-A1-2013/030223
- US-A1- 2009 005 598

## Beschreibung

Gegenstand der Erfindung ist ein Verfahren zur Herstellung von Toluylendiamin durch Hydrierung von Dinitrotoluol in Gegenwart eines Katalysators, wobei als Ausgangsmaterial ein Dinitrotoluol eingesetzt wird, das durch Anlegen einer elektrischen Spannung gemäß Anspruch 1 behandelt wurde.

Toluylendiamine sind Zwischenprodukte für die Herstellung von Toluylendiisocyanaten (TDI), die wichtige, im großtechnischen Maßstab hergestellte Vorprodukte zur Herstellung von Polyurethanen sind. Ihre Herstellung durch katalytische Hydrierung von Dinitrotoluolen ist bekannt und vielfach beschrieben (Ullmann's Enzyklopädie der technischen Chemie, 4.Auflage, Band 7, Seite 393 ff, 1973, Verlag Chemie Weinheim / New York). Die industrielle Herstellung der Toluylendiamine erfolgt vorwiegend durch Umsetzung eines durch Nitrierung von Toluol mit Salpetersäure zugänglichen Gemisches isomerer Dinitrotoluole (nachfolgend auch DNT). Handelsübliche Gemische isomerer Dinitrotoluole werden vorwiegend mit Salpetersäure in Gegenwart von Schwefelsäure als Katalysator in einem zweistufigen isothermen Nitrierprozess unter intermediärer Bildung der entsprechenden Mononitrotoluole als Roh-DNT hergestellt. Sie werden anschließend in der Reaktion nachgeschalteten Stufen, vorwiegend in Wäschen, aufbereitet und so weitgehend von gelösten Schwefel- und Salpetersäuregehalten wie auch von in den Reaktionsstufen gebildeten Nebenkomponenten, z. B. Kresolen und deren Abbauprodukten, befreit.

Die katalytische Hydrierung handelsüblicher DNT-Produkte kann unter Mitverwendung eines inerten Lösungsmittels oder in Substanz erfolgen, wobei die Gemische dann vor Durchführung der Hydrierung aufgeschmolzen werden. Sie kann sowohl diskontinuierlich als auch kontinuierlich unter Verwendung der üblichen Reaktoren durchgeführt werden. Für den wirtschaftlichen Erfolg des angewandten Verfahrens wesentlich sind neben einer kontinuierlichen Reaktionsführung vor allem die mit dem angewandten Verfahren erzielbaren Selektivitäten der Umsetzung sowie die Kapazitäten und Standzeiten der eingesetzten Katalysatoren.

US-B-3 356 728 offenbart ein verbessertes kontinuierliches Verfahren zur Herstellung von aromatischen Aminen durch katalytische Hydrierung aromatischer Polynitroaromaten in einem Schlammphasenreaktor, wobei das Verfahren beispielhaft an der Umsetzung von Dinitrotoluol erläutert wird. Nach der Lehre von US-B-3 356 728 erfolgt die katalytische Hydrierung von Dinitrotoluol in diesem Reaktionssystem im Hinblick auf Selektivität, Katalysatorstandzeit und Durchsatz besonders effektiv, wenn
▪ die Reaktionszone bei der Umsetzung stets mit Wasserstoff gesättigt ist,
▪ die aromatische Polynitroverbindung dem System unter Einhaltung eines bestimmten Gewichtsverhältnisses zu dem im Reaktionssystem vorhandenen Katalysator zugesetzt wird ("Katalysatorbeladung") und
▪ die Konzentration der zugesetzten aromatischen Polynitroverbindung in der Reaktionszone einen angegebenen Grenzwert nicht übersteigt.

Nach der Lehre von US-B- 3 356 728 führen die beanspruchten Katalysatorbeladungen zu hohen Konzentrationen an aktivem Katalysator im Reaktionssystem, so dass die eingespeiste aromatische Polynitroverbindung nach ihrem Eintritt in das Gemisch sofort zu dem gewünschten Amin umgesetzt wird, dadurch die Konzentration an unreduzierter Nitroverbindung in dem Reaktionssystem zu jeder Zeit kleiner 0,005 Gew.-% gehalten wird. Nach der Lehre von US-B-3 356 728 wird durch diese geringe Konzentration eine schnelle Vergiftung des Katalysators verhindert, werden darüber hinaus bei der Umsetzung der aromatischen Polynitroverbindung höhere Ausbeuten und eine verbesserte Produktreinheit bei wesentlich reduzierten Kosten erhalten. Die Schrift offenbart nicht, dass durch eine besondere Behandlung unter Anlegen einer elektrischen Spannung des zu hydrierenden DNT der Katalysatorverbrauch reduziert werden kann.

Mit der Verbesserung der Stoffaustauschvorgänge bei heterogen katalysierten Hydrierreaktionen, wie zum Beispiel der Herstellung aromatischer Amine aus den entsprechenden Nitroverbindungen, beschäftigt sich WO 02/062729 A2**.** Durch einen bestimmten Inertgasanteil im Hydrierwasserstoff soll eine höhere Wasserstoffsättigung der Flüssigphase im Reaktor erreicht werden, was einer erhöhten Alterung des Hydrierkatalysators und einer unzureichenden Selektivität der Reaktion vorbeugen soll. Ziel auch dieser Veröffentlichung ist es letztendlich, für eine möglichst vollständige Umsetzung der Nitroverbindung zum Amin im Hydrierreaktor zu sorgen.

EP 1 935 870 A1 offenbart ein Verfahren zur Herstellung von Toluylendiamin, bei dem Verbesserungen der Katalysatorstandzeiten und Selektivität der Umsetzung durch eine Minimierung des Kohlendioxidgehalts des in der Umsetzung eingesetzten Dinitrotoluols erzielt werden. Dies wird insbesondere durch Eintragen eines Inertgases zumindest in der letzten Stufe der mehrstufigen Extraktion des rohen Dinitrotoluols mit Wasser erreicht.

WO 2013/030223 A1 befasst sich mit dem Problem, dass in Nitrobenzol enthaltende Verunreinigungen bei dessen Hydrierung zu Anilin in der Gasphase zu Problemen in der Betriebsstabilität und/oder der Katalysatoraktivität führen können, wenn das Nitrobenzol mittels Verdüsung in die Gasphase überführt wird. Zur Lösung dieses Problem wird ein verbessertes Verfahren zur Herstellung von Nitrobenzol vorgeschlagen, das sich insbesondere durch einen Waschschritt unter Verwendung einer wässrigen Lösung eines Kaliumsalzes, gefolgt von einer Neutralwäsche, auszeichnet. In bevorzugten Ausgestaltungen wird in der abschließenden Neutralwäsche eine Elektrophorese zur Brechung der in der Wäsche erhaltenen Dispersion eingesetzt.

Es bestand ein Bedarf an Verfahren, den Katalysatorverbrauch in der Hydrierung weiter zu senken.

Überraschenderweise wurde nun gefunden, dass bei der Herstellung von Toluylendiamin, bei der Dinitrotoluol mit Wasserstoff in Gegenwart eines Katalysators umgesetzt wird, der Katalysatorverbrauch reduziert werden kann, wenn man als Ausgangsmaterial ein Dinitrotoluol einsetzt, das durch Anlegen einer elektrischen Spannung gemäß Patentanspruch 1 behandelt wurde. Die erfindungsgemäße Behandlung führt zu einem DNT, das sich bei dessen anschließender Hydrierung vorteilhaft auswirkt.

Ein Gegenstand der vorliegenden Erfindung ist daher ein Verfahren zur Herstellung von Toluylendiamin durch Hydrierung eines durch Anlegen einer elektrischen Spannung gemäß Anspruch 1 behandelten Dinitrotoluol-Ausgangsmaterials in Gegenwart eines Katalysators.

Der Begriff *Toluylendiamin (TDA)* umfasst dabei alle Isomere. Bevorzugt werden mit dem erfindungsgemäßen Verfahren TDA-Isomerengemische produziert, die - nach Destillation-78 Massen-% bis 82 Massen-% 2,4-TDA und 18 Massen-% bis 22 Massen-% 2,6 TDA, jeweils bezogen auf die Gesamtmasse aller TDA-Isomeren, enthalten. 2,4-TDA und 2,6-TDA werden in der Literatur auch als meta-TDA bezeichnet.

Nachstehend werden verschiedene Ausführungsformen des erfindungsgemäßen Verfahrens erläutert. Diese können beliebig miteinander kombiniert werden, sofern sich aus dem Zusammenhang nichts anderes ergibt.

In einer bevorzugten Ausgestaltung des erfindungsgemäßen Verfahrens enthält das durch Anlegen einer elektrischen Spannung behandelte Dinitrotoluol-Ausgangsmaterial 90 Massen-% bis 99,5 Massen-% Dinitrotoluol (DNT) und 0,5 Massen-% bis 10 Massen-% Wasser.

Die Behandlung des DNT durch Anlegen einer elektrischen Spannung erfolgt erfindungsgemäß durch Anlegen einer Gleichspannung von 50 Volt bis 1000 Volt in einer Zelle mit einem Elektrodenabstand von 1 mm bis 100 mm, wobei das Dinitrotoluol-Ausgangsmaterial der Gleichspannung für einen Zeitraum von 0,01 Sekunden bis 100 Sekunden, bevorzugt 0,1 Sekunden bis 10 Sekunden ausgesetzt wird. Dabei stellt sich ein Strom von 0,1 Ampere bis 10 Ampere ein.

In einer weiteren bevorzugten Ausgestaltung umfasst das erfindungsgemäße Verfahren die folgenden Schritte:
(i) Dinitrierung von Toluol zu Dinitrotoluol;
(ii) Aufarbeitung des in Schritt (i) erhaltenen rohen Verfahrensproduktes durch (ii.1) Abtrennung der wässrigen Phase und (ii.2) anschließende mindestens einstufige Wäsche der in (ii.1) erhaltenen Dinitrotoluol-Phase mit mindestens einem wässrigen Waschmedium, wobei jede Waschstufe Vermischung der Dinitrotoluol-Phase mit wässrigem Waschmedium und anschließende Abtrennung wässrigen Waschmediums umfasst;
(iii) Behandlung des Dinitrotoluols durch Anlegen einer elektrischen Spannung, wobei diese Behandlung vor oder nach Abtrennung in Schritt (ii) eingesetzten wässrigen Waschmediums erfolgen kann;
(iv) Hydrierung des durch Anlegen einer elektrischen Spannung behandelten Dinitrotoluol-Ausgangsmaterials.

Dabei kann die Dinitrierung des Toluols in **Schritt (i)** grundsätzlich nach allen aus dem Stand der Technik bekannten Verfahren erfolgen. Bevorzugt erfolgt die Dinitrierung mit einem Gemisch aus Schwefel- und Salpetersäure (Nitriersäure). Neben dem einstufigen Prozess (EP-A-0 908 442) ist eine zweistufige Nitrierung das allgemein gängige und auch im Rahmen der vorliegenden Erfindung bevorzugte Verfahren. Im zweistufigen Verfahren wird zunächst Toluol mit Salpetersäure und Schwefelsäure zum Mononitrotoluol (MNT) umgesetzt (Monostufe). Nach Trennung des entstandenen Reaktionsgemisches in MNT und eine Säurephase, was in statischen Abscheidern oder in dynamischen Abscheidern ausgeführt werden kann, wird das MNT erneut mit Salpetersäure und Schwefelsäure zum Dinitrotoluol (DNT) umgesetzt (Di-Stufe). Die Schwefelsäurephase aus der Monostufe wird aufkonzentriert. In der Di-Stufe wird aufkonzentrierte Schwefelsäure eingesetzt. Das Reaktionsgemisch der Di-Stufe wird in eine organische Phase, das Roh-DNT, und eine Säurephase getrennt, wobei die Säurephase als Einsatz-Schwefelsäure der Mono-Stufe dienen kann oder einer Aufkonzentrierung zugeführt wird. Die Trennung dieses Reaktionsgemisches der Di-Stufe kann ebenfalls in statischen oder dynamischen Abscheidern erfolgen.

In einer besonders bevorzugten Ausgestaltung wird das zweistufige Verfahren wie folgt durchgeführt:
(i.1) Toluol wird mit Nitriersäure unter Einhaltung eines Massenverhältnisses von wässriger zur organischer Phase von > 2, bevorzugt > 3, ganz besonders bevorzugt > 3,5 und unter Dispergierung der organischen Phase in die wässrige Phase zu Mononitrotoluol umgesetzt, wobei ein Mononitrotoluol enthaltendes Reaktionsgemisch erhalten wird;
(i.2) das Mononitrotoluol enthaltende Reaktionsgemisch wird in eine Mononitrotoluol enthaltende organische Phase und eine Schwefelsäure enthaltende wässrige Phase aufgetrennt;
(i.3) die Mononitrotoluol enthaltende organische Phase wird mit Nitriersäure unter Einhaltung eines Massenverhältnisses von wässriger zur organischer Phase von > 1,5 bevorzugt > 3, ganz besonders bevorzugt > 3,5 und unter Dispergierung der organischen Phase in die wässrige Phase umgesetzt, wobei ein Dinitrotoluol enthaltendes Reaktionsgemisch erhalten wird;
(i.4) das Dinitrotoluol enthaltende Reaktionsgemisch wird in eine Dinitrotoluol enthaltende organische Phase und eine Schwefelsäure enthaltende wässrige Phase aufgetrennt;
wobei insgesamt pro mol Toluol weniger als 2,06 mol an Salpetersäure eingesetzt werden.

In den Schritten (i.1) und (i.3) wird bevorzugt der Eintrag an Misch- bzw. Dispergierenergie so gewählt, dass einerseits die gewünschten Dispersionen mit sehr großen Phasengrenzflächen erzeugt werden und andererseits eine Bildung stabiler "Tropfen in Tropfen"-Emulsionen vermieden wird. Die in die Reaktionsschritten vorteilhaft einzubringende Misch- bzw. Dispergierenergie kann in einfachen Versuchen leicht ermittelt werden. Auch bei Einhaltung der erfindungsgemäßen Phasenverhältnisse ist sie abhängig von der gewählten Reaktor- und Rührergeometrie sowie den physikalischen Daten der Reaktionsgemische. Einmal eingestellt, kann der Zustand der in den Reaktionssystemen vorliegenden Dispersionen jedoch vorteilhaft über die Leitfähigkeit der homogen durchmischten Reaktionsmischungen kontrolliert werden.

Bevorzugt wird dabei die Umsetzung in Schritt (i.1) und / oder in Schritt (i.3) isotherm durchgeführt.

In einer bevorzugten Ausführungsform erfolgt die Umsetzung in den Reaktionsschritten (i.1) und (i.3) jeweils in Kaskaden von Reaktoren, in denen eine Vermischung stattfindet, besonders bevorzugt in Kaskaden mit jeweils 2 bis ≤ 4 Reaktoren. In einer besonders bevorzugten Form werden als Reaktoren Schleifenreaktoren eingesetzt, die Umwälzpumpen und Wärmeaustauscher umfassen.

In einer ganz besonders bevorzugten Form werden in der Mononitrierstufe zwei in Reihe geschaltete Schleifenreaktoren, die Umwälzpumpen und Wärmeaustauscher enthalten, eingesetzt. In der Dinitrierstufe werden dann zwei in Reihe geschaltete Schleifenreaktoren, die Umwälzpumpe und Wärmeaustauscher umfassen, sowie zusätzlich ein weiterer Schleifenreaktor, der eine Umwälzpumpe, aber keinen Wärmeaustauscher umfasst, eingesetzt. Dabei erfolgt die Dimensionierung der Umwälzpumpen so, dass stets eine Dispersion der organischen in der homogenen wässrigen Phase erfolgt. Bevorzugt erfolgt in dieser Ausführungsform in wenigstens einem der eingesetzten Reaktoren zusätzlich eine Leitfähigkeitsmessung zur kontinuierlichen Kontrolle des Zustands der umgewälzten Dispersion.

Den Reaktionsschritten (i.1) und (i.3) ist jeweils ein Phasentrennschritt (i.2) und (i.4) nachgeschaltet. Dabei können alle zur Phasentrennung geeignete Apparate zum Einsatz kommen. Es sind sowohl dynamisch wie auch statische Abscheider geeignet. In einer bevorzugten Ausführungsform kommen auf beiden Stufen (Schritte (i.2) und (i.4)) statische Abscheider zum Einsatz.

In der bevorzugten Ausführungsform wird in Schritt (i.1) das Toluol der Mononitrierstufe zugeführt. Bevorzugt erfolgt die Zufuhr des Toluols in den ersten Reaktor, ein Split der Toluolzugabe auf mehrere Reaktoren ist aber ebenfalls möglich. Bevorzugt wird das in den ersten Reaktor eingespeiste Toluol über eine oder mehrere Düsen mit der Nitriersäure vermischt dem Reaktor zugegeben. Dabei wird bevorzugt vorab die Schwefelsäure enthaltende wässrige Phase aus Schritt (i.4) (Absäure der Dinitrierstufe) mit Salpetersäure vermischt und so die Nitriersäure hergestellt. Es kann aber auch frische Schwefelsäure oder ein Gemisch aus frischer Schwefelsäure und der Absäure aus der Dinitrierstufe eingesetzt werden.

Eine getrennte Zugabe des Toluols sowie der Nitriersäure sind ebenfalls möglich. Die getrennte Zugabe der Salpetersäure sowie der Schwefelsäure, z. B. der Absäure aus der Dinitrierstufe, sind ebenfalls möglich. Das Massenverhältnis der Phasen in den Reaktoren der Mononitrierstufe wird bevorzugt über die Menge der in die Reaktoren eingespeiste Schwefelsäure, z. B. der Absäure der Dinitrierstufe, eingestellt. Aber auch eine Rückführung der in Schritt (i.2) erhaltenen Schwefelsäure enthaltenden wässrigen Phase aus der Mononitrierstufe ist möglich, wobei die rückgeführte Schwefelsäure enthaltende wässrige Phase bevorzugt auf den ersten Reaktor der Stufe gespeist wird. Eine Zugabe auf mehrere Reaktoren der Stufe ist jedoch ebenfalls möglich. Auch ein Split der der Mononitrierung zugeführten Salpetersäure auf mehrere Reaktoren ist möglich.

Die Umsetzung des Toluols erfolgt auf der Mononitrierstufe (Schritt (i.1)) bevorzugt in einem Temperaturbereich von 30 °C bis 70 °C, wobei die Reaktoren der Mononitrierstufe bei gleicher Reaktionstemperatur betrieben werden können. Dem Reaktionsfortschritt angepasste, unterschiedliche Reaktionstemperaturen in den einzelnen Reaktoren sind jedoch ebenfalls möglich.

Bevorzugt werden die in den Reaktoren der Mononitrierstufe (Schritt (i.1)) vorliegenden Dispersionen mit Hilfe von Leitfähigkeitsmessungen kontinuierlich überwacht. So werden unzulässige Abweichungen, die beispielsweise erkennbar sind an einem signifikanten Abfall der Leitfähigkeit der umgewälzten Reaktionsgemische, durch Änderungen der über die Misch- bzw. Dispergiereinrichtungen in die Reaktoren eingetragene Mischenergie oder aber bevorzugt über eine Änderung des Phasenverhältnisses in den Reaktoren der Nitrierstufe korrigiert.

Bei der vorstehend beschriebenen Ausgestaltung von Schritt (i) wird ein sehr hoher Toluolumsatz mit einer sehr effektiven Ausnutzung der in die Mononitrierstufe eingespeisten Salpetersäure erreicht. Die in der nachfolgende Phasentrennung gewonnene Schwefelsäure enthaltende wässrige Phase (Absäure) weist typischerweise Salpetersäuregehalte von < 0,1 Massen-%, bezogen auf die Gesamtmasse der Absäure, auf. Diese niedrigen Gehalte führen zu dem sehr niedrigen spezifischen Salpetersäurebedarf dieses Verfahrens. Zusätzlich verringern sie den zur Aufkonzentrierung der Absäure der Mononitrierstufe notwendigen Aufwand.

In der bevorzugten Ausgestaltung von Schritt (i) wird die in der Phasentrennung der Mononitrierstufe (Schritt (i.2)) erhaltene organische Phase bevorzugt ohne weitere Aufarbeitung den Reaktoren der Dinitrierstufe (Schritt (i.3)) zugeführt. Bevorzugt erfolgt die Zufuhr der MNT enthaltenden organischen Phase in den ersten Reaktor, ein Split auf mehrere Reaktoren ist aber ebenfalls möglich. Bevorzugt wird das in den ersten Reaktor eingespeiste MNT über eine oder mehrere Düsen mit der Nitriersäure vermischt dem Reaktor zugegeben. Dabei wird bevorzugt vorab die Schwefelsäure enthaltende wässrige Phase aus Schritt (i.2) (Absäure der Mononitrierstufe) ggf. aufbereitet und mit Salpetersäure vermischt und so die Nitriersäure hergestellt. Es kann aber auch frische Schwefelsäure oder ein Gemisch aus frischer Schwefelsäure und der Absäure aus der Mononitrierstufe eingesetzt werden. Eine getrennte Zugabe der MNT enthaltenden organischen Phase aus Schritt (i.2) sowie der Nitriersäure sind ebenfalls möglich. Die getrennte Zugabe der Salpetersäure sowie der Schwefelsäure, z. B. der aufbereiteten Absäure aus der Mononitrierstufe, sind ebenfalls möglich. Das Gewichts-Verhältnis der Phasen in den Reaktoren der Dinitrierstufe (Schritt (i.3)) wird bevorzugt über die Menge der in die Reaktoren eingespeiste Schwefelsäure, z.B. der aufbereiteten Absäure aus der Mononitrierstufe, eingestellt. Aber auch eine Rückführung der in Schritt (i.4) erhaltenen Schwefelsäure enthaltenden wässrigen Phase aus der Dinitrierstufe ist möglich, wobei die rückgeführte Schwefelsäure enthaltende wässrige Phase bevorzugt auf den ersten Reaktor der Stufe gespeist wird. Eine Zugabe auf mehrere Reaktoren der Stufe ist jedoch ebenfalls möglich. Auch ein Split der der Dinitrierung zugeführten Salpetersäure auf mehrere Reaktoren ist möglich.

Die Umsetzung des Mononitrotoluols erfolgt auf der Dinitrierstufe (Schritt (i.3)) in einem Temperaturbereich von 55 °C bis 80 °C, wobei die Reaktoren der Dinitrierstufe bei gleicher Reaktionstemperatur betrieben werden können. Dem Reaktionsfortschritt angepasste, unterschiedliche Reaktionstemperaturen in den einzelnen Reaktoren sind jedoch ebenfalls möglich.

Bevorzugt werden die in den Reaktoren der Dinitrierstufe (Schritt (i.3)) vorliegenden Dispersionen mit Hilfe von Leitfähigkeitsmessungen kontinuierlich überwacht. So werden unzulässige Abweichungen, die beispielsweise erkennbar sind an einem signifikanten Abfall der Leitfähigkeit der umgewälzten Reaktionsgemische, durch Änderungen der über die Misch- bzw. Dispergiereinrichtungen in die Reaktoren eingetragene Mischenergie oder aber bevorzugt über eine Änderung des Phasenverhältnisses in den Reaktoren der Nitrierstufe korrigiert.

Bei der bevorzugten Ausgestaltung von Schritt (i) wird ein sehr hoher Mononitrotoluolumsatz mit einer sehr effektiven Ausnutzung der in die Dinitrierstufe eingespeisten Salpetersäure erreicht. Die in der nachfolgenden Phasentrennung gewonnene Schwefelsäure enthaltende wässrige Phase (Absäure) weist typischerweise Salpetersäuregehalte von < 0,2 Massen-%, bezogen auf die Gesamtmasse der Absäure, auf. Die Beladungen des in Schritt (i.4) erhaltenen DNT enthaltenden organischen Phase mit Salpetersäure liegen dabei bei < 0,4 Massen-% HNO₃, bezogen auf die Masse der organischen Phase. Diese Ausgestaltung des Schritts (i) ist in EP -A-1 880 989 beschrieben.

In allen Herstellverfahren von DNT durch Toluolnitrierung mit Nitriersäure fällt ein rohes Verfahrensprodukt an, das in einem weiteren **Schritt (ii)** aufgearbeitet wird. Hierzu wird zunächst die wässrige Phase abgetrennt **(Schritt (ii.1),** was in dem Fachmann bekannten Phasentrennapparaturen geschehen kann. Dabei verbleibt eine organische, DNT enthaltende Phase (im Folgenden auch als Roh-DNT bezeichnet), die, abhängig von den genauen Reaktions- und Trennbedingungen, noch bis zu 5 Massen-%, bevorzugt bis zu 1,2 Massen-% Salpetersäure und bis zu 6 Massen-%, bevorzugt bis zu 1,5 Massen-% Schwefelsäure enthalten kann. Daneben sind noch Nebenprodukte der Nitrierung in einer Konzentration von bis zu ca. 1 Massen-% vorhanden. Die Nebenprodukte sind im Wesentlichen Nitrokresole, Pikrinsäure und Nitrobenzoesäuren.

Dieses durch einfache Phasentrennung erhaltene Roh-DNT kann direkt in Schritt (ii.2) gewaschen werden. Es ist jedoch auch möglich und insbesondere bei hohen Restgehalten an Säuren bevorzugt, aus diesem Roh-DNT zuvor noch eine wässrige, Schwefel- und Salpetersäure enthaltende Phase abzutrennen. Zu diesem Zweck wird das durch einfache Phasentrennung erhaltene Roh-DNT mit bis zu 10 Massen-% Wasser, bevorzugt mit 0,3 Massen-% bis 8 Massen-%, bezogen auf die Gesamtmasse des Roh-DNT, bevorzugt bei einer Temperatur von 65 °C bis 95 °C, vermischt. Dabei scheidet sich eine wässrige, Schwefel- und Salpetersäure enthaltende Phase ab, die abgetrennt wird. Es ist auch möglich, diesen Vorgang zweistufig durchzuführen, indem man das durch einfache Phasentrennung erhaltene Roh-DNT zunächst mit 0,3 Massen-% bis 3 Massen-% Wasser, bezogen auf die Gesamtmasse des Roh-DNT, vermischt, die sich abscheidende, überwiegend Schwefelsäure enthaltende wässrige Phase abtrennt, und im Anschluss daran die verbleibende organische Phase mit 2 Massen-% bis 6 Massen-% Wassern, bezogen auf die Gesamtmasse dieser organischen Phase, vermischt, und die sich abscheidende überwiegend Salpetersäure enthaltende Phase abtrennt. Diese Ausgestaltung des Schritts (ii.1) ist in EP -A-0 279 312 beschrieben.

Die in Schritt (ii.1) erhaltene, DNT umfassende Phase wird in **Schritt (ii.2)** einer Wäsche mit mindestens einem wässrigen Waschmedium unterzogen. Diese Wäsche kann grundsätzlich so erfolgen wie aus dem Stand der Technik bekannt.

In einer bevorzugten Ausgestaltung wird die DNT umfassende Phase in zwei bis vier Waschstufen mit einem wässrigen Waschmedium von Säuren und Nebenkomponenten befreit. Das in diesem Prozess zugeführte wässrige Waschmedium kann in mindestens einer Waschstufe eine Base enthalten, wobei üblicherweise Natronlauge oder Natriumcarbonat in Konzentrationen von 2 bis 10 Massen-% eingesetzt wird. Bevorzugt wird die Base nicht in der letzten Waschstufe eingesetzt, d. h. für die letzte Waschstufe wird bevorzugt neutrales Wasser verwendet. Während durch die neutrale Wasserwäsche weitgehend Schwefelsäure und Salpetersäure aus dem Nitrierprodukt entfernt werden, überführt die alkalische Wäsche auch salzbildende organische Komponenten wie zum Beispiel Nitrokresole, Pikrinsäure und Nitrobenzoesäuren in die wässrige Phase.

Als wässriges Waschmedium kann, außer in der letzten Waschstufe, im Gegenstrom geführtes Waschwasser einer Folgestufe eingesetzt werden. Das eingesetzte Waschwasser kann aber auch Frischwasser, demineralisiertes Wasser oder Wasser einer geeigneten Qualität aus einem Folgeprozess des beschriebenen Nitrierverfahrens sein.

Die eingesetzten Mengen an Waschwasser betragen bevorzugt 15 bis 90 Gew.-Teile, besonders bevorzugt 50 bis 65 Gew.-Teile Waschwasser pro 100 Gew.-Teile DNT.

Die Wäsche in Schritt (ii.2) umfasst besonders bevorzugt drei bis vier Stufen. Bevorzugt wird dabei in der ersten Waschstufe als wässriges Waschmedium neutrales oder leicht saures Wasser (z. B. aus wässrigen Rezyklatströmen des Nitrierprozesses oder der zugehörigen Schwefelsäureaufkonzentrierung) eingesetzt. In dieser Waschstufe entsteht je nach eingesetzter Waschwassermenge und Waschwasserführung ein saures Prozessabwasser mit bevorzugten Säuregehalten von 1,0 bis 3,0 Massen-% Salpetersäure und 2,0 bis 6,0 Massen-% Schwefelsäure sowie einem DNT Gehalt von einigen 1000 ppm. Die Konzentration an organischen Nebenprodukten der Nitrierung liegt im genannten Prozessabwasser im Allgemeinen zwischen 300 und 900 ppm. In der zweiten Waschstufe wird bevorzugt ein mit einer Base (bevorzugt Natriumhydroxid oder Natriumcarbonat) alkalisch gestelltes Waschwasser eingesetzt. Im Abwasserstrom dieser alkalischen Wäsche sind im Allgemeinen 3,0 bis 7,0 Massen-% organische Nebenprodukte der Nitrierung, im Wesentlichen Nitrokresole, Pikrinsäure und Nitrobenzoesäuren, in Form ihrer wasserlöslichen Salze der eingesetzten Base enthalten. Daneben kann dieser Abwasserstrom auch noch mehrere 1000 ppm DNT sowie 2,0 bis 4,0 Massen-% Salpetersäure und ca. 0,6 bis 1,2 Massen-% Schwefelsäure in Form ihrer wasserlöslichen Salze enthalten. Der Abwasserstrom der alkalischen Wäsche hat einen pH-Wert > 7,0, vorzugsweise >7,5 (gemessen bei 80° C). An diese alkalische Wäsche schließen sich bevorzugt ein bis zwei Waschstufen mit neutralem Wasser an.

In einer anderen Ausführungsform des Schrittes (ii.2) wird auf den Zusatz einer Base verzichtet, wobei dann die Wäsche in 2 bis 6 Stufen durchgeführt wird.

In allen Ausführungsformen wird in der letzten Waschstufe bevorzugt neutrales Wasser eingesetzt.

Die Waschstufen werden in dem Fachmann bekannten geeigneten Apparaten, vorzugsweise in Wasch- bzw. Extraktionskolonnen oder in Mischer-Scheider Apparaturen durchgeführt.

Die Wäsche in Schritt (ii.2) ist bevorzugt als mehrstufige Extraktion umfassend Vermischung und Phasentrennung in jeder Stufe ausgestaltet. Bei dieser bevorzugten Ausführungsform erfolgt die Extraktion in den Stufen als "Flüssig/Flüssig"-Extraktion. Dies wird durch eine geeignete Wahl der Temperaturen der eingesetzten DNT umfassenden Phase wie auch der als Extraktionsmittel eingesetzten wässrigen Phasen sichergestellt.

In einer weiteren bevorzugten Ausführungsform erfolgt die mehrstufige Extraktion zumindest auf einer Stufe unter Einsatz eines Apparates zum Mischen und Trennen praktisch nicht ineinander lösbarer Flüssigkeiten unterschiedlichen spezifischen Gewichtes, wie er z. B. in DE-B-1 135 425 beschrieben ist.

Kennzeichen des in DE-B-1 135 425 beschrieben Apparates sind eine als Extraktions- bzw. Waschkolonne ausgebildete Mischzone mit einem konzentrisch um die Mischzone angeordnetem Raum zur Phasentrennung, in den das die Mischzone verlassende Gemisch über ein Überlaufwehr mit umgebenden Hohlmantel der "abgeschnittenen" Waschkolonne eintritt und nach Maßgabe der Dichten in zwei Phasen aufgetrennt wird. Um den in DE-B-1 135 425 in Spalte 2 / Zeilen 35-52 und Spalte 3/Zeilen 1-12 in seinem Aufbau sowie in Spalte 3/Zeilen 29-47 in seiner Funktion beschrieben Apparat in dem erfindungsgemäßen Verfahren vorteilhaft einzusetzen, sollte in dem Apparat zusätzlich noch eine Möglichkeit für die Zufuhr von Inertgas (bevorzugt Stickstoff) in die Mischzone, beispielsweise durch eine zusätzliche Öffnung im Bereich des Bodens dieses Apparateabschnitts, eingerichtet werden.

Es können für die Wäsche in Schritt (ii.2) aber grundsätzlich jede Form von mehrstufigen Extraktionsverfahren und Extraktionsapparaten umfassend Vermischung und Phasentrennung in jeder Stufe eingesetzt werden, wobei die Verweilzeit der Vermischung jeder Stufe der Extraktion in Schritt (ii.2) bevorzugt mindestens 4 Minuten und höchstens 60 Minuten beträgt, und bevorzugt zumindest in der letzten Stufe der Extraktion in Schritt (ii.2) in die Vermischung (d. h. in das Volumen, in dem die Vermischung stattfindet) zusätzlich ein Inertgas (bevorzugt Stickstoff) eingetragen wird.

In **Schritt (iii)** erfolgt die Behandlung der in Schritt (ii) erhaltenen gewaschenen DNT enthaltenden Phase durch Anlegen einer elektrischen Spannung. Dies erfolgt durch Anlegen einer Gleichspannung von 50 Volt bis 1000 Volt in einer Zelle mit einem Elektrodenenabstand von 1 mm bis 100 mm, wobei das Dinitrotoluol-Ausgangsmaterial der Gleichspannung für einen Zeitraum von 0,01 Sekunden bis 100 Sekunden, bevorzugt 0,1 Sekunden bis 10 Sekunden ausgesetzt wird. Dabei stellt sich ein Strom von 0,1 bis 10 Ampere ein. Bevorzugt erfolgt diese Behandlung mittels einer Elektrokoaleszenz-Vorrichtung. Im Rahmen der vorliegenden Erfindung umfasst der Begriff *Elektrokoaleszenz-Vorrichtung* einen Apparat, in welchem das gewaschene DNT durch eine Zelle strömt, deren Elektroden mit einer Spannungsquelle verbunden sind. Die Zelle kann beispielsweise mittels platten- oder zylinderförmigen Elektroden ausgeführt werden. Im Falle von plattenförmigen Elektroden wählt man üblicherweise mindestens zwei rechtwinklige Elektroden, die parallel zueinander angeordnet sind. Werden mehr als zwei Elektroden eingesetzt, wird man diese zweckmäßigerweise abwechselnd mit der Spannungsquelle verbinden. Die Elektrodenanordnung wird man in einem Apparat oder Behälter einsetzen, wobei eine der Elektroden auch die Behälterwand selbst sein kann. Beispiele für geeignete Elektrokoaleszenz-Vorrichtungen sind die in EP-A-0 004 278 und EP-A-1 816 117 beschriebenen "Elektrophorese-Einheiten".

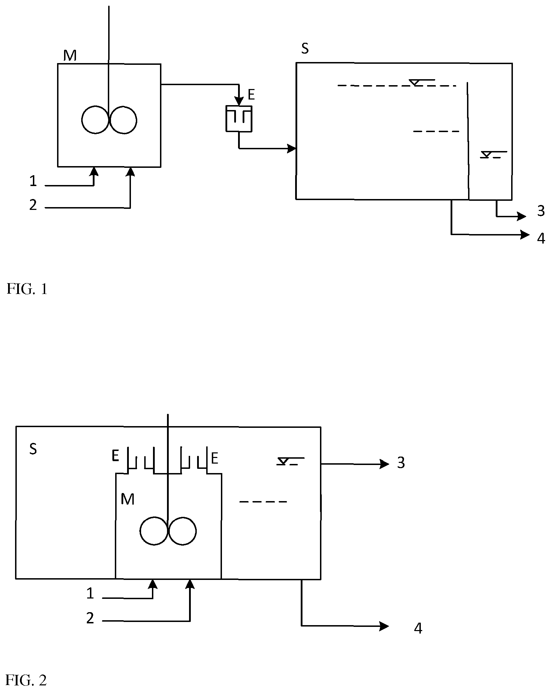

Die erfindungsgemäße Behandlung des DNT durch Anlegen einer elektrischen Spannung gemäß Anspruch 1 erfolgt bevorzugt in oder nach der letzten Waschstufe von Schritt (ii.2). Dies kann so geschehen, dass der Vermischung von Dinitrotoluol und wässrigem Waschmedium in der letzten Waschstufe von Schritt (ii.2) (welche bevorzugt mit neutralem, d. h. weder basisch noch sauer gestelltem Wasser durchgeführt wird) eine Elektrokoaleszenz-Vorrichtung zur Behandlung durch Anlegen einer elektrischen Spannung in Schritt (iii) nachgeschaltet ist. In der letzten Waschstufe kann dabei ein statischer oder dynamischer (bevorzugt umfassend einen oder mehrere Rührer oder eine Pumpe, besonders bevorzugt eine Kreiselpumpe) Mischer eingesetzt werden. Bevorzugt wird in dieser Ausgestaltung des Schritts (ii.2) ein dynamischer Mischer eingesetzt, der bevorzugt mit einem oder mehreren Rührern versehen ist. Die Vermischung mittels eines Rührers hat den Vorteil, dass auch bei kleineren Durchsätzen eine ausreichende Mischleistung erzielt wird. Diese Ausgestaltung ist in FIG. 1 dargestellt. Dabei ist 1 der DNT-Strom, 2 ein Strom von zugeführtem Waschwasser, 3 das abgeführte Waschwasser und 4 das von Verunreinigungen befreite DNT. M bezeichnet die Mischeinrichtung, E die Elektrokoaleszenz-Vorrichtung und S den Phasentrennapparat. In M wird das bevorzugt bereits in vorangegangenen Stufen des Schritts (ii) gewaschene DNT 1 mit (bevorzugt neutralem) Waschwasser 2 vermischt. Das erhaltene DNT-Wasser-Gemisch durchströmt die Elektrokoaleszenz-Vorrichtung E, bevor es im Phasentrennapparat S in eine wässrige Phase 3 (abgeführtes Waschwasser) und eine organische Phase 4 (das durch Anlegen einer elektrischen Spannung behandelte DNT-Ausgangsmaterial) getrennt wird. Die in FIG. 1 gezeigte Anordnung ist natürlich auch bei Einsatz eines statischen Mischers realisierbar. Zur Implementierung des erfindungsgemäßen Verfahrens in eine vorhandene DNT-Produktionsanlage kann der Mischer der vorhandenen letzten Waschstufe als Mischer M eingesetzt werden. Es ist jedoch ebenfalls möglich, der vorhandenen letzten Waschstufe eine Anordnung wie in FIG. 1 gezeigt als kombinierte Wasch- und Elektrokoaleszenz-Einrichtung nachzuschalten.

Es ist ebenfalls möglich, die die Elektrokoaleszenz-Vorrichtung in die Vermischung von DNT-Phase und wässrigem Waschmedium der letzten Waschstufe des Schritts (ii.2) zu integrieren, d. h. Elektrokoaleszenz-Vorrichtung im Mischer der letzten Waschstufe anzuordnen. In einer bevorzugten Ausgestaltung dieser Ausführungsform sind eine Mischzone, eine Elektrokoaleszenz-Vorrichtung und eine Absetzzone in einem Apparat, der in mehrere Zonen unterteilt ist, vereint. Dabei befindet sich die Mischzone, in welcher DNT und Waschwasser vermischt werden, bevorzugt innen, und die Absetzzone, in welcher die wässrige und die DNT-Phase getrennt werden, bevorzugt außen. Die Vermischung von DNT und Waschwasser kann mittels dynamischer (z. B. ein oder mehrere Rührer, eine Pumpe, bevorzugt eine Kreiselpumpe) oder statischer Mischer erfolgen. Dynamische Mischer sind auch in dieser Ausführungsform bevorzugt. FIG. 2 zeigt eine entsprechende Anordnung.

Es ist auch möglich, die Elektrokoaleszenz-Vorrichtung in einem Absetztank für DNT, welcher dem Phasentrennapparat der letzten Waschstufe in Schritt (ii.2) (welche bevorzugt mit neutralem, d. h. weder basisch noch alkalisch gestelltem Wasser durchgeführt wird) zur Aufnahme des gewaschenen, vom Waschwasser getrennten DNT nachgeschaltet ist, oder in einer Rohrleitung, welche den Phasentrennapparat der letzten Waschstufe mit dem Absetztank verbindet, einzubauen. Insbesondere wenn die Elektrokoaleszenz-Vorrichtung im Absetztank angeordnet ist, kann es vorteilhaft sein, dem Absetztank zusätzlich zur gewaschenen Dinitrotoluol umfassenden Phase Wasser zuzuführen, und dem Absetztank, bevorzugt diskontinuierlich, eine sich abscheidende wässrige Phase zu entnehmen. Auf diese Weise gelingt es, die Hydrierung evtl. störende Nebenprodukte mit der wässrigen Phase auszutragen.

Auch bei Anordnung der Elektrokoaleszenz-Vorrichtung im Absetztank oder einer die letzte Waschstufe mit dem Absetztank verbindenden Rohrleitung ist es bevorzugt, in der letzten Waschstufe von Schritt (ii.2) einen dynamischen Mischer einzusetzen.

In jedem Fall kann der Vorgang der Trennung von DNT und Wasser durch dem Fachmann geläufige geeignete Einbauten in den Phasentrennapparat wie beispielsweise Platten, Gestricke oder poröse Formkörper wie beispielsweise Filterkerzen unterstützt werden.

Das in Schritt (iii) erhaltene durch Anlegen einer elektrischen Spannung behandelte DNT-Ausgangsmaterials wird in **Schritt (iv)** zu Toluylendiamin (TDA) hydriert. Diese Hydrierung kann grundsätzlich nach jedem aus dem Stand der Technik bekannten Verfahren erfolgen. Bevorzugt wird wie folgt verfahren:
Das in Schritt (iii) erhaltene durch Anlegen einer elektrischen Spannung behandelten DNT-Ausgangsmaterial wird bevorzugt in einer Vorlage gesammelt und aus dieser in flüssiger Form der bevorzugt kontinuierlich betriebenen Hydrierung zugeführt. Die der Vorlage bevorzugt kontinuierlich zufließenden Dinitrotoluole können dabei sowohl vor der Sammlung in der Vorlage, in der Vorlage oder nach Entnahme aus der Vorlage einer Strippgasbehandlung mit einem Inertgas unterzogen werden. Die katalytische Hydrierung des DNT-Ausgangsmaterials kann unter Mitverwendung eines inerten Lösungsmittels oder in Substanz erfolgen. Sie erfolgt bevorzugt in Substanz unter Einsatz einer wässrigen Katalysatorsuspension. Sie kann sowohl diskontinuierlich als auch kontinuierlich unter Verwendung der üblichen Reaktoren durchgeführt werden. Beispiele hierfür sind Rührkessel, Blasensäulen, oder Schlaufenreaktoren, wie Loop-Venturi-Reaktoren, oder Strahlschlaufenreaktoren mit innerem und äußerem Kreislauf.

Die Wasserstoffzufuhr in das System erfolgt dabei bevorzugt so, dass stets der stöchiometrische Bedarf an Wasserstoff für die Umsetzung der eingespeisten Nitrogruppenäquivalente zu den entsprechenden Aminverbindungen gedeckt wird und zusätzlich der Reaktorinhalt unter besonderer Berücksichtigung der Oberflächen des eingesetzten Katalysators stets mit Wasserstoff gesättigt ist.

Als Katalysatoren können in Schritt (iv) alle zur katalytischen Hydrierung von aromatischen Nitroverbindungen bekannten Hydrierkatalysatoren eingesetzt werden. Gut geeignet sind insbesondere die Metalle der 8. Nebengruppe des Periodensystems der Elemente oder Mischungen derselben sowie Kupfer. Besonders bevorzugt ist der Katalysator ausgewählt aus Katalysatoren enthaltend Pt, Pd, Rh, Ru, Ni, Co, Cu oder deren Mischungen; dabei wird der Katalysator in einer Konzentration von 0,01 Massen-% bis < 20 Massen-%, bezogen auf die Gesamtmasse des Reaktionsgemisches bei der Hydrierung, eingesetzt. Die Katalysatoren können beispielsweise auf Trägermaterialien wie Kohlenstoff oder Oxiden von Magnesium, Aluminium und/oder Silizium aufgebracht sein. In einer weiteren Ausgestaltung des erfindungsgemäßen Verfahrens werden Raney-Eisen, -Kobalt und/oder -Nickel, insbesondere Nickel-haltige Katalysatoren wie beispielsweise Raney-Nickel-Katalysatoren, sowie Palladium- oder Platin-haltige Katalysatoren auf Trägermaterialien eingesetzt, deren Herstellung und Anwendung als Hydrierkatalysator von aromatischen Nitroverbindungen wie z. B. Nitrobenzol, Nitrotoluolen, Dinitrotoluolen, chlorierten Nitroaromaten und anderen bekannt ist und bereits öfters beschrieben wurde (EP 0 223 035 B1, EP1 066 111 B1, EP-A-1 512 459).

In einer ganz besonders bevorzugt Ausführung des erfindungsgemäßen Verfahrens werden als Katalysatoren Raney-Nickel-Katalysatoren eingesetzt, wie sie in EP-A-1 512 459 beschrieben sind. Diese Katalysatoren zeichnen sich im Vergleich zu herkömmlichen Raney-Nickel-Katalysatoren durch eine deutlich erhöhte Produkt-Selektivität und Katalysatorstandzeit insbesondere bei Reaktionstemperaturen von > 120 °C. Durch ihren Einsatz kann mithin die bei der Hydrierung von Dinitrotoluolen freiwerden Reaktionswärme besonders vorteilhaft zur Erzeugung von als Heizmedium einsetzbarem Wasserdampf genutzt werden.

Dem bevorzugt kontinuierlich betriebenen Reaktionssystem zur katalytischen Hydrierung der Dinitrotoluole wird das Reaktionsgemisch nach Maßgabe der Speisungen entnommen, bevorzugt kontinuierlich unter Rückhaltung des Katalysators im System. Besonders bevorzugt erfolgt die Entnahme unter Einsatz einer Querstromfiltration, wie sie z.B. in EP 0 634 391 B1 im Prinzip oder in EP 1 137 623 B1 in einer speziellen Ausführungsform beschrieben sind. Bei diesem Produktaustrag wird dem Reaktor ein Teilstrom entnommen, dieser über ein Querstromfilter geführt, dort dem Produktstrom ein Teilmenge unter Rückhaltung des Katalysators entnommen, abschließend der verminderte, bezüglich seines Katalysatorgehalts "aufkonzentrierte" Teilstrom wieder dem Reaktor zugeführt.

Desaktivierter Katalysator kann durch frischen Katalysator ersetzt werden, ohne dass dafür notwendigerweise die Produktion unterbrochen werden müsste. Bei einem ausreichend dimensionierten Reaktor ist es möglich, über einen langen Zeitraum frischen Katalysator zuzuführen, ohne verbrauchten Katalysator zu entfernen. Es ist natürlich auch möglich, die Reaktion so auszugestalten, dass verbrauchter Katalysator kontinuierlich oder periodisch ausgetragen wird, sodass theoretisch ohne Unterbrechung produziert werden kann. In jedem Fall zeichnet sich das erfindungsgemäße Verfahren durch einen geringen Katalysatorverbrauch aus.

Das filtrierte Produkt weist eine hohe Reinheit auf und kann ohne weitere chemische Nachbehandlung nach dem Stand der Technik zu dem Endprodukt Toluylendiamin (TDA) aufbereitet werden. Nach dem Stand der Technik umfasst diese Aufbereitung die Abtrennung von Reaktionswasser sowie, sofern verwendet, von Lösungsmittel, und die Reinigung des TDA auf eine für die gewünschte Folgeanwendung - in der Regel (und auch im Rahmen der vorliegenden Erfindung bevorzugt) die Phosgenierung zu Toluylendiisocyanat (TDI) - erforderliche Reinheit. Das Reaktionswasser wird von leichtflüchtigen organischen Verunreinigungen gereinigt. Eine solche Aufbereitung des nach Filtration erhaltenen TDA-haltigen Verfahrensprodukt kann so ausgestaltet sein, dass zunächst Reaktionswasser destillativ abgetrennt wird, wobei der in diesem Schritt erhaltene TDA-Strom und der in diesem Schritt erhaltene Wasser-Strom in nachgelagerten Destillationsschritten weiter aufgereinigt werden. Es ist ebenfalls möglich, wie EP-A-0 236 839 offenbart, Abtrennung und Reinigung des Reaktionswassers in einem Schritt durchzuführen. In jedem Fall wird ein Strom abgetrennten und destillativ gereinigten Reaktionswassers erhalten, der entweder als Abwasser entsorgt oder einer anderen Verwendung zugeführt werden kann. EP-A-1 484 312 offenbart die Verwendung solchen Wassers in der Wäsche von rohem DNT. Auch im Rahmen der vorliegenden Erfindung ist es möglich, abgetrenntes und destillativ gereinigtes Reaktionswasser aus der DNT-Hydrierung (Schritt (iv)) in der Wäsche von Schritt (ii.2) einzusetzen. Dies gilt auch für die letzte Waschstufe von Schritt (ii.2), in oder nach welcher in einer bevorzugten Ausgestaltung des erfindungsgemäßen Verfahrens die elektrische Behandlung (iii) gemäß Anspruch 1 stattfindet.

### Beispiele

### Beispiel 1 (Vergleich)

In einem kontinuierlich betriebenen Reaktor zur Hydrierung von DNT wird DNT technischer Reinheit in Gegenwart eines Ni-basierten Katalysators in einem Massenanteil von 7 %, bezogen auf den Reaktorinhalt, und einem Druck von 24 bar (absolut) bei einer Verweilzeit von 1,8 h hydriert. Zur Aufrechterhaltung der Reaktion ist es erforderlich, im Mittel alle 15 h Katalysator zuzugeben.

### Beispiel 2 (erfindungsgemäß)

Es wird eine Einheit wie in FIG. 1 gezeigt in Betrieb genommen (mit der Ausnahme, dass ein Statikmischer anstelle des gezeigten Mischers mit Rührers eingesetzt wird). Dabei werden 50 kg Wasser (2) pro t DNT (1) in einem Statikmischer M gemischt. Das erhaltene DNT-Wasser-Gemisch wird in einer Elektrokoaleszens-Vorrichtung E bei 400 V für 1 Sekunde behandelt, und das erhaltene behandelte Gemisch wird in einem Trennapparat S von Wasser (3) getrennt. Das DNT (4) wird in dem gleichen Reaktor, der auch in Beispiel 1 eingesetzt wurde, unter den gleichen Bedingungen von Druck, Katalysatorart und -konzentration sowie Verweilzeit hydriert. Jetzt ist die Zugabe von neuem Katalysator nur noch im Mittel alle 31 h erforderlich.

## Patentansprüche

1. Verfahren zur Herstellung von Toluylendiamin durch Hydrierung eines durch Anlegen einer elektrischen Spannung behandelten Dinitrotoluol-Ausgangsmaterials in Gegenwart eines Katalysators, wobei als elektrische Spannung eine Gleichspannung von 50 Volt bis 1000 Volt eingesetzt wird, der das Dinitrotoluol-Ausgangsmaterial in einer Zelle mit einem Elektrodenenabstand von 1 mm bis 100 mm für einen Zeitraum von 0,01 Sekunden bis 100 Sekunden ausgesetzt wird.

2. Verfahren nach Anspruch 1, bei dem das durch Anlegen einer elektrischen Spannung behandelte Dinitrotoluol-Ausgangsmaterial 90 Massen-% bis 99,5 Massen-% Dinitrotoluol und 0,5 Massen-% bis 10 Massen-% Wasser enthält.

3. Verfahren nach einem der Ansprüche 1 bis 2, bei dem das Verfahren die folgenden Schritte umfasst:
(i) Dinitrierung von Toluol zu Dinitrotoluol;
(ii) Aufarbeitung des in Schritt (i) erhaltenen rohen Verfahrensproduktes durch (ii.1) Abtrennung der wässrigen Phase und (ii.2) anschließende mindestens einstufige Wäsche der in (ii.1) erhaltenen Dinitrotoluol-Phase mit mindestens einem wässrigen Waschmedium, wobei jede Waschstufe Vermischung der Dinitrotoluol-Phase mit wässrigem Waschmedium und anschließende Abtrennung wässrigen Waschmediums umfasst;
(iii) Behandlung des Dinitrotoluols durch Anlegen einer elektrischen Spannung, wobei diese Behandlung vor oder nach Abtrennung in Schritt (ii) eingesetzten wässrigen Waschmediums erfolgen kann;
(iv) Hydrierung des durch Anlegen einer elektrischen Spannung behandelten Dinitrotoluol-Ausgangsmaterials.

4. Verfahren nach Anspruch 3, bei dem die Wäsche in Schritt (ii.2) zwei bis vier Stufen umfasst und das wässrige Waschmedium mindestens einer der Stufenaußer der letzten Stufe eine Base enthält.

5. Verfahren nach Anspruch 3, bei dem die Wäsche in Schritt (ii.2) 2 bis 6 Stufen umfasst und das wässrige Waschmedium in keiner der Stufen eine Base enthält.

6. Verfahren nach einem der Ansprüche 3 bis 5, bei dem die Behandlung in Schritt (iii) durch Anlegen einer elektrischen Spannung in oder nach der letzten Waschstufe in Schritt (ii.2) durchgeführt wird.

7. Verfahren nach Anspruch 6, bei dem der Vermischung von Dinitrotoluol und wässrigem Waschmedium in der letzten Waschstufe von Schritt (ii.2) eine Elektrokoaleszenz-Vorrichtung zur Behandlung des Dinitrotoluols durch Anlegen einer elektrischen Spannung in Schritt (iii) nachgeschaltet ist.

8. Verfahren nach Anspruch 6, bei dem eine Elektrokoaleszenz-Vorrichtung zur Behandlung des Dinitrotoluols durch Anlegen einer elektrischen Spannung in Schritt (iii) in die Vermischung von Dinitrotoluol-Phase und wässrigem Waschmedium der letzten Waschstufe von Schritt (ii.2) integriert ist.

9. Verfahren nach Anspruch 6, bei dem die Abtrennung wässrigen Waschmediums in der letzten Waschstufe von Schritt (ii.2) in einem Phasentrennapparat mit diesem nachgeschaltetem Absetztank zur Aufnahme der gewaschenen Dinitrotoluol-Phase durchgeführt wird, wobei der Absetztank oder eine den Absetztank mit dem Phasentrennapparat verbindende Rohrleitung mit einer Elektrokoaleszenz-Vorrichtung zur Behandlung durch Anlegen einer elektrischen Spannung in Schritt (iii) versehen ist.

10. Verfahren nach Anspruch 9, bei dem dem Absetztank zusätzlich zur gewaschenen Dinitrotoluol-Phase Wasser zugeführt wird, und bei dem dem Absetztank die sich absetzende wässrige Phase kontinuierlich oder diskontinuierlich entnommen wird.

11. Verfahren nach Anspruch 10, bei dem die Entnahme der wässrigen Phase diskontinuierlich erfolgt.

12. Verfahren nach einem der Ansprüche 1 bis 11, bei dem der Katalysator ausgewählt ist aus Katalysatoren enthaltend Pt, Pd, Rh, Ru, Ni, Co, Cu oder deren Mischungen, und bei dem der Katalysator in einer Konzentration von 0,01 Massen-% bis < 20 Massen-%, bezogen auf die Gesamtmasse des Reaktionsgemisches bei der Hydrierung, eingesetzt wird.

## Claims

1. Process for preparing toluenediamine by hydrogenation in the presence of a catalyst of a dinitrotoluene starting material which has been treated by application of an electric potential, where a DC voltage of from 50 volt to 1000 volt is used as electric potential to which the dinitrotoluene starting material is subjected in a cell having an electrode spacing of from 1 mm to 100 mm for a period of from 0.01 second to 100 seconds.

2. Process according to Claim 1, wherein the dinitrotoluene starting material treated by application of an electric potential contains from 90% by mass to 99.5% by mass of dinitrotoluene and from 0.5% by mass to 10% by mass of water.

3. Process according to any of Claims 1 to 2, wherein the process comprises the following steps:
(i) dinitration of toluene to form dinitrotoluene;
(ii) work-up of the crude process product obtained in step (i) by (ii.1) removal of the aqueous phase and (ii.2) subsequent at least single-stage scrubbing of the dinitrotoluene phase obtained in (ii.1) by means of at least one aqueous scrubbing medium, where each scrubbing stage comprises mixing of the dinitrotoluene phase with aqueous scrubbing medium and subsequent removal of the aqueous scrubbing medium;
(iii) treatment of the dinitrotoluene by application of an electric potential, where this treatment can be carried out before or after removal of aqueous scrubbing medium used in step (ii);
(iv) hydrogenation of the dinitrotoluene starting material treated by application of an electric potential.

4. Process according to Claim 3, wherein the scrub in step (ii.2) comprises from two to four stages and the aqueous scrubbing medium of at least one of the stages apart from the last stage contains a base.

5. Process according to Claim 3, wherein the scrub in step (ii.2) comprises from 2 to 6 stages and the aqueous scrubbing medium does not contain a base in any of the stages.

6. Process according to any of Claims 3 to 5, wherein the treatment in step (iii) is carried out by application of an electric potential in or after the last scrubbing stage in step (ii.2).

7. Process according to Claim 6, wherein the mixing of dinitrotoluene and aqueous scrubbing medium in the last scrubbing stage of step (ii.2) is followed by an electrocoalescence apparatus for treatment of the dinitrotoluene by application of an electric potential in step (iii).

8. Process according to Claim 6, wherein an electrocoalescence apparatus for treatment of the dinitrotoluene by application of an electric potential in step (iii) is integrated into the mixing of dinitrotoluene phase and aqueous scrubbing medium in the last scrubbing stage of step (ii.2).

9. Process according to Claim 6, wherein the removal of aqueous scrubbing medium in the last scrubbing stage of step (ii.2) is carried out in a phase separation apparatus having said downstream settling tank for taking up the scrubbed dinitrotoluene phase, where the settling tank or a pipe connecting the settling tank to the phase separation apparatus is provided with an electrocoalescence apparatus for the treatment by application of an electric potential in step (iii).

10. Process according to Claim 9, wherein water is fed in addition to the scrubbed dinitrotoluene phase into the settling tank and the aqueous phase which settles out is taken off continuously or discontinuously from the settling tank.

11. Process according to Claim 10, wherein the aqueous phase is taken off discontinuously.

12. Process according to any of Claims 1 to 11, wherein the catalyst is selected from among catalysts containing Pt, Pd, Rh, Ru, Ni, Co, Cu or mixtures thereof and the catalyst is used in a concentration of from 0.01% by mass to < 20% by mass, based on the total mass of the reaction mixture in the hydrogenation.

## Revendications

1. Procédé de fabrication de toluylène-diamine par hydrogénation d'un matériau de départ dinitrotoluène traité par application d'une tension électrique en présence d'un catalyseur, une tension continue de 50 volt à 1 000 volt étant utilisée en tant que tension électrique, à laquelle le matériau de départ dinitrotoluène est exposé dans une cellule ayant un écart entre les électrodes de 1 mm à 100 mm pendant une durée de 0,01 seconde à 100 secondes.

2. Procédé selon la revendication 1, selon lequel le matériau de départ dinitrotoluène traité par application d'une tension électrique contient 90 % en masse à 99,5 % en masse de dinitrotoluène et 0,5 % en masse à 10 % en masse d'eau.

3. Procédé selon l'une quelconque des revendications 1 à 2, selon lequel le procédé comprend les étapes suivantes :
(i) la dinitration de toluène en dinitrotoluène ;
(ii) le traitement du produit de procédé brut obtenu à l'étape (i) par (ii.1) séparation de la phase aqueuse, puis (ii.2) lavage en au moins une étape de la phase dinitrotoluène obtenue en (ii.1) avec au moins un milieu de lavage aqueux, chaque étape de lavage comprenant un mélange de la phase dinitrotoluène avec un milieu de lavage aqueux, puis la séparation du milieu de lavage aqueux ;
(iii) le traitement du dinitrotoluène par application d'une tension électrique, ce traitement pouvant avoir lieu avant ou après la séparation du milieu de lavage aqueux utilisé à l'étape (ii) ;
(iv) l'hydrogénation du matériau de départ dinitrotoluène traité par application d'une tension électrique.

4. Procédé selon la revendication 3, selon lequel le lavage à l'étape (ii.2) comprend deux à quatre étapes et le milieu de lavage aqueux d'au moins une des étapes à l'exception de la dernière étape contient une base.

5. Procédé selon la revendication 3, selon lequel le lavage à l'étape (ii.2) comprend 2 à 6 étapes et le milieu de lavage aqueux ne contient une base dans aucune des étapes.

6. Procédé selon l'une quelconque des revendications 3 à 5, selon lequel le traitement à l'étape (iii) est réalisé par application d'une tension électrique lors de ou après la dernière étape de lavage à l'étape (ii.2).

7. Procédé selon la revendication 6, selon lequel un dispositif à électro-coalescence pour le traitement du dinitrotoluène par application d'une tension électrique à l'étape (iii) est raccordé en aval du mélange du dinitrotoluène et du milieu de lavage aqueux lors de la dernière étape de lavage de l'étape (ii.2).

8. Procédé selon la revendication 6, selon lequel un dispositif à électro-coalescence pour le traitement du dinitrotoluène par application d'une tension électrique à l'étape (iii) est intégré dans le mélange de la phase dinitrotoluène et du milieu de lavage aqueux de la dernière étape de lavage de l'étape (ii.2).

9. Procédé selon la revendication 6, selon lequel la séparation du milieu de lavage aqueux lors de la dernière étape de lavage de l'étape (ii.2) est réalisée dans un appareil de séparation de phases comprenant une cuve de décantation raccordée en aval de celui-ci pour la réception de la phase dinitrotoluène lavée, la cuve de décantation ou une canalisation raccordant la cuve de décantation avec l'appareil de séparation de phases étant munie d'un dispositif à électro-coalescence pour le traitement par application d'une tension électrique à l'étape (iii).

10. Procédé selon la revendication 9, selon lequel de l'eau est introduite dans la cuve de décantation en plus de la phase dinitrotoluène lavée, et selon lequel la phase aqueuse qui se dépose est soutirée de manière continue ou discontinue de la cuve de décantation.

11. Procédé selon la revendication 10, selon lequel le soutirage de la phase aqueuse a lieu de manière discontinue.

12. Procédé selon l'une quelconque des revendications 1 à 11, selon lequel le catalyseur est choisi parmi les catalyseurs contenant Pt, Pd, Rh, Ru, Ni, Co, Cu ou leurs mélanges, et selon lequel le catalyseur est utilisé en une concentration de 0,01 % en masse à < 20 % en masse, par rapport à la masse totale du mélange réactionnel lors de l'hydrogénation.
